(19) Europäisches Patentamt
European Patent Office
Office européen des brevets

(11) Publication number: **0 168 989 A1**

(12) **EUROPEAN PATENT APPLICATION**

(21) Application number: **85304437.8**

(22) Date of filing: **20.06.85**

(51) Int. Cl.[4]: **C 07 C 43/13**, C 07 C 41/01, B 01 J 31/20

(30) Priority: **21.06.84 US 622815**
**21.06.84 US 622816**

(43) Date of publication of application: **22.01.86**
**Bulletin 86/4**

(84) Designated Contracting States: **BE DE FR GB IT NL**

(71) Applicant: **Sun Refining and Marketing Company, 1801 Market Street, Philadelphia, PA 19103 (US)**

(72) Inventor: **Lyons, James E., 211 Cooper Drive, Wallingford, PA. 19086 (US)**
Inventor: **Duggan, D. Michael, 311 South Darlington Street, West Chester, PA. 19380 (US)**
Inventor: **Myers, Harry K., Jr., Limestone Road, Cochranville, PA. 19330 (US)**

(74) Representative: **Lewin, John Harvey et al, Elkington and Fife High Holborn House 52/54 High Holborn, London WC1V 6SH (GB)**

(54) **Rutheniumpromoted cobalt catalysts for the dealkoxyhydroxymethylation of formaldehyde acetals to form glycol ethers.**

(57) Ruthenium carbonyl-promoted cobalt carbonyl catalysts effectively catalyze the dealkoxyhydroxymethylation of formaldehyde acetals, wherein said acetal may be prepared separately or in situ from formaldehyde and an alcohol. Methylal, for example, may be reacted with syngas, i.e., CO and $H_2$, in the presence of this ruthenium carbonyl-promoted cobalt catalyst to form the monomethyl ether of ethylene glycol. Cycloalkyl and aryl-formaldehyde acetals, as well as alkyl acetals, may likewise be reacted with syngas in the presence of this ruthenium carbonyl-cobalt carbonyl catalyst to form the corresponding ethylene glycol monoether.

The invention also is directed to the ruthenium carbonyl-promoted cobalt carbonyl catalysts per se, including novel precipitated forms of these ruthenium-cobalt catalyst mixtures.

EP 0 168 989 A1

Ruthenium-Promoted Cobalt Catalysts for the Dealkoxyhydroxymethylation of Formaldehyde Acetals to Form Glycol Ethers

This invention relates to the dealkoxyhydroxymethylation of acetals. More particularly, it relates to a novel process for the dealkoxyhydroxymethylation of certain dialkyl-, dicycloalkyl-, or diaryl-formaldehyde acetals by reacting said acetals with syngas, i.e., hydrogen and carbon monoxide, in the presence of novel ruthenium carbonyl-promoted cobalt carbonyl catalysts to form the corresponding ethylene glycol monoethers. Still more particularly, it relates to the catalysts per se and methods for preparing the same. The methylal described herein may be added directly or formed *in situ* from the corresponding formaldehyde and alcohol precurors.

The glycol ethers described herein encompass known classes of compounds having various uses, as for example as jet fuel additives, cleaners, coatings solvents, intermediates in the production of certain diphthalates, and the like.

One current well-known method of manufacturing ethylene glycol monoethers such as monoalkyl ethers consists of reacting ethylene oxide with the alcohol corresponding to the desired alkyl ether, employing various known catalyst systems.

Alternatively, the cobalt-catalyzed reaction of aldehydes or their dialkyl acetals with syngas, i.e., a carbon monoxide-hydrogen mixture, to form the corresponding glycol ether is also described in the art. Thus, for example, a method of making ethylene glycol ethers is described in U.S. Patent No. 2,525,793 which employs cobalt oxide to catalyze the reaction of methylal with syngas to provide a reaction mixture which, after hydrogenation over nickel, gives relatively uneconomical conversions on the order of 25-33%.

Numerous attempts have been made to obtain more practical yields of glycol ethers from aldehydes or their dialkylacetals. A number of promoters have been used in conjunction with various cobalt catalysts in an effort to improve reaction rates and product yields. U.S. Patent 4,062,898, for example, discloses a ruthenium chloride-promoted cobalt iodide catalyst which hydrocarbonylates formaldehyde dimethylacetal (methylal) to ethylene glycol monomethyl ether (EGMME) in yields of 10% or less. The reaction temperature required is 185°C at 20 atm. or above. A second method, described in Jpn. Kokai Tokkyo Koho 81 83,432 (1981) uses substantial quantities of 2,4,6- collidine or similar aromatic amines to promote the cobalt carbonyl-catalyzed hydrocarbonylation of methylal in benzene as a solvent. The reaction of methylal with highly pressurized syngas in this process at 190°C for 10 hours gave 44% selectivity to EGMME at 98% conversion. A further patent, Euro. Pat. Appln. EP 34,374 (1981) uses both iodine and triphenyl or tricyclohexylphosphine together with $RuCl_3 \cdot H_2O$ to promote the $Co(Ac)_2 \cdot 4H_2O$ - catalyzed hydrocarbonylation of methylal using 3000 psig

of syngas, and temperatures of between 150 and 175°C to obtain results nearly comparable to those of the Japanese.

More recently, Knifton has found that cobalt carbonyl promoted with a Group VIB donor ligand catalyzes the hydrocarbonylation of an aldehyde in an alcohol to make ethylene glycol monoethers; U.S. 4,308,403. Yields of ethylene glycol monobutyl ether (EGMBE) as high as 61% were reported in this patent. A cyclopentadienyl-ligated cobalt catalyst is also effective for these reactions giving glycol ethers in up to 54% yield; U.S. 4,317,943.

Propylene glycol monoalkyl ethers are formed by contacting high pressure mixtures of carbon monoxide and hydrogen with either an acetal or an aldehyde and an alcohol using a cobalt catalyst promoted with a tin- or germanium-containing compound; U.S. 4,356,327. Yields of glycol ethers up to 31% were reported in this patent. Ethylene glycol ethers were also formed from a formaldehyde acetal or formaldehyde and an alcohol using tin or germanium promoters for cobalt carbonyl; U.S. 4,357,477. The highest glycol ether yield (EGMBE) was 53% in this case.

Finally, propylene glycol monoalkyl ethers were formed by hydrocarbonylation of acetaldehyde acetals or acetaldehyde and alcohols using rhodium, ruthenium or nickel compounds to promote either cobalt carbonyls or cobalt compounds having group V ligand systems attached. Glycol ether yields up to 28% were realized when these promoters were used; Knifton, U.S. 4,390,734 (1983).

Thus, the use of various promoters for the cobalt-catalyzed hydrocarbonylation of aldehydes or acetals has resulted in glycol ether yields of from 10-61%, depending on the glycol ether produced. The highest reported yield of EGMME is 44%, of EGMBE is 61% and PGMEE is 28%.

In accordance with the present invention, there is provided an improved process for the reaction of certain dialkyl-, dicycloalkyl-, or diaryl-formaldehyde acetals or their formaldehyde-alcohol precursors with syngas in the presence of novel ruthenium carbonyl-promoted cobalt carbonyl catalysts, to form the corresponding ethylene glycol monoethers. This reaction, which may best be described as the dealkoxyhydroxymethylation of an acetal formed separately or in situ by the known reaction of formaldehyde with an alcohol, may be depicted by the following general reaction scheme.

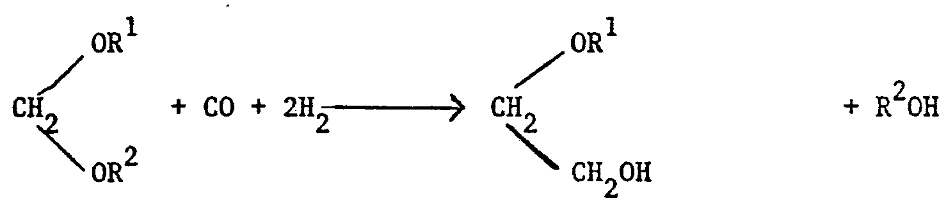

wherein $R^1$ and $R^2$, which may be the same or different, and which together may be joined by one or more bridging atoms as described below to form a cyclic compound, comprise any organic moieties which are inert to the conditions of the reaction, and are selected from the group consisting of:

a) a straight or branched chain alkyl group having from 1 to about 20 carbon atoms, such as methyl, ethyl, propyl, isopropyl, n-butyl, t-butyl, 2-ethylhexyl, dodecyl, and the like;

b) a substituted or unsubstituted cycloalkyl group having from 1 to about 20 carbon atoms, such as cyclopentyl, cyclohexyl, cycloheptyl, 3-methylcyclopentyl, 3-butylcyclohexyl, cyclooctyl, adamantyl, decalyl, 3-phenylcycloheptyl and the like; and

c) a substituted or unsubstituted aryl group having from 1 to about 20 carbon atoms such as benzyl, phenyl, naphthyl, fluoroanthryl, tetralyl, tolyl, ethylphenyl, cumyl, anisyl, chlorophenyl, and the like.

It will be understood that when $R^1$ and $R^2$ in the foregoing reaction scheme are different, the resulting products will actually be mixtures of the corresponding ethers and alcohols.

This invention is also described to the novel ruthenium-promoted cobalt carbonyl catalysts per se, and to methods for preparing the same.

In a further embodiment it has been found that additionally there may be employed as the catalyst a composition comprising a ruthenium carbonyl-promoted cobalt carbonyl metal cluster of the formula $Co_2 Ru(CO)_{11}$, which may be prepared by the method disclosed by Roland et al, Angew. Chem. Int. Ed. Engl., 20, 679 (1981), and which is useful for preparing glycol ethers derived from other acetals as well, such as diethoxymethane, dileutoxymethane, diethoxyethane and the like.

This process, using the novel catalysts of this invention, provides an improvement over the methods of the prior art in that the instant catalysts do not require the added presence of the iodide, amines, or phosphine promoters such as are disclosed in the prior art, and thus are less costly and easier to prepare and recover. Moreover, these novel catalysts permit the reaction to be carried out under more mild conditions of time and temperature than those of the prior art, yet most surprisingly provide rates and selectivities of desired product over those obtained by the use of cobalt carbonyl alone.

The novel homogenous catalysts of this invention which may be prepared in varying ways, are ruthenium carbonyl-promoted dicobalt octacarbonyls, more specifically, triruthenium dodecacarbonyl-dicobalt octacarbonyl mixtures. This catalyst system is readily prepared by simply mixing dicobalt octacarbonyl ($Co_2(CO)_8$) with ruthenium dodecacarbonyl ($Ru_3(CO)_{12}$) in the reaction medium. The molar ratios of these two components are optimally in the range of about 10:1 to 1:10, and preferably about 5:1 to 1:5.

It has also been found, in accordance with this invention, that when the catalyst mixture is allowed to precipitate from the reaction medium following completion of the hydroxymethylation reaction this material unexpectedly has been found to have superior catalytic activity over the initial ruthenium-cobalt carbonyl mixture in terms of rates and selectivities. This activity has been found to vary somewhat, however,

depending upon the rate at which the precipitate forms, along with other factors such as the initial concentrations, ratios, and the like. Thus, for example, when the ruthenium and cobalt carbonyl catalysts are mixed into the reaction medium in molar ratios of 1:1, and the mixture allowed to precipitate out of solution, following dealkoxyhydroxymethylation, for a period of 1-25 days, the resulting solid, when introduced into a methylal-syngas reaction, has equal or increased activity with respect to rates and selectivities for ethylene glycol monoether.

Equally surprising, it has also been found that an even more active catalyst than the precipitate described above may be obtained by heating the ruthenium and cobalt mixture for periods of from about 1 to 5 hours in the presence of an inert organic solvent, preferably a chlorinated aromatic such as chlorobenzene, o-dichlorobenzene, or the like, under pressurized syngas. The mixture, on cooling, results in an orange-colored precipitate, which when used as a catalyst in the dealkoxylhydroxymethylation of methylal, results in significant improvements in the rates and selectivities for the ethylene glycol ether over the original catalyst mixture. For example, when ruthenium and cobalt carbonyl are mixed in molar ratios of 1:1, heated in chlorobenzene at about 150° C. under ~3000 psi of syngas for about 3 hours, and then cooled, the resulting precipitate has increased catalytic activity in a methylal dealkoxyhydroxymethylation reaction.

The formaldehyde acetal dealkoxyhydroxymethylation reaction with syngas, as described above, utilizing the novel ruthenium-cobalt carbonyl catalyst mixtures of this invention, including the aforedescribed

precipitated forms thereof, may conveniently be conducted in a generally known manner whereby the desired acetal is reacted with syngas under elevated temperature and pressures for given periods of time, during which period the reaction mixture is efficiently stirred. In this reaction, the volume ratio of carbon monoxide to hydrogen in the syngas desirably is in the range of from about 1:3 to 3:1, and more preferably 1:2 to 2:1. Following rapid cooling, the reaction product is then recovered from the mixture in a routine manner.

In contrast to the prior art reaction conditions described above, the novel ruthenium-cobalt carbonyl catalysts of this invention advantageously permit the use of more mild operating conditions. Thus, temperatures in the range of from about 100 to 200°C, and preferably about 125 to 175°C, pressures of from about 500 to 5000 psi, and preferably about 1000 to 3000 psi, may satisfactorily be employed.

The ratio of the weight of catalyst mixture employed, to the weight of acetal, is desirably in the range of from about .001:0.1, and preferably .005:.05 in a batch reaction.

In a further embodiment of this invention, it has been found that highly advantageous effects may also be obtained in this dealkoxyhydroxymethylation process by the addition of solvents in combination with the catalyst system of this invention.

The solvents which may be advantageously used comprise any polar or non-polar organic solvents which are inert to the conditions of the

reaction. Included amongst these solvents are $C_{1-12}$ alcohols, preferably those corresponding to the alkyl group of the formaldehyde acetal, such as methanol, ethanol, butanol, 3-ethyl-2-hexanol and the like; ethers which will not cleave under the conditions of the reaction, such as glyme, diglyme, diphenyl ether and the like; aromatics and substituted aromatics such as benzene, toluene, xylene, chlorobenzene, dichlorobenzene, anisole, and the like.

The solvents may constitute anywhere from 0 to 90 volume percent of the reaction mixture, and preferably 20 to 80 percent.

The acetal starting materials employed in this invention have the aforedescribed general formula, namely

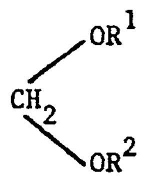

wherein the $R^1$ and $R^2$ groups are as defined above. These acetals can be prepared in a known manner, separately or in situ, as for example as described in E.V. Dehmlav and J. Schmidt, Tetrahedron Letters, p.95-6 (1976) B.S. Bal and H.W. Pinnick, J. Org. Chem. V44 (21), p. 3727-8(1979) D.W. Hall, U.S. Patent 3,492,356, Jan. 27 (1970), by the reaction of formaldehyde with an alcohol, or mixture of alcohols, of the general formula $R^1OH$ or $R^2OH$, where again $R^1$ and $R^2$ are as defined above, form the corresponding acetal. Hereinafter, when the acetal is referred to, it will be understood that the corresponding precursors, formaldehyde and the desired alcohol, are also intended to be included.

As also mentioned above, the organic substituents of the resulting acetal may be joined by one or more bridging atoms to form such cyclic compounds as

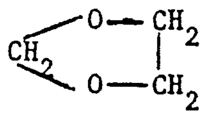 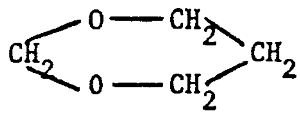 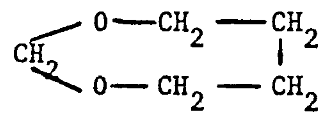

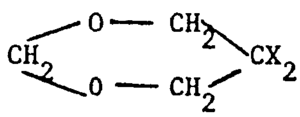, 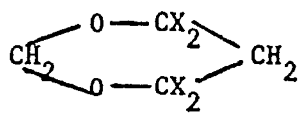

and the like, wherein X is selected from the group consisting of alkyl, aralkyl, aryl and cycloalkyl groups having from 1 to about 20 carbon atoms.

It is essential, in selecting the acetal starting material, that it not contain any substituents which are reactive under the conditions of the dealkoxyhydroxymethylation process of this invention. In other words, the $R^1$ and $R^2$ groups should not, for example, contain or comprise such reactive moieties as phosphine, arsine, amino, sulfido or carbonyl groups, acetal moieties, or olefins or acetylenic double bonds. Other like groups will be recognized or readily determined by those skilled in the art as resulting in products other than the desired monoethers.

When these acetals are dealkoxyhydroxymethylated with syngas in accordance with the process of this invention, there is obtained the corresponding ethylene glycol monoether in which the ether moiety will correspond to the organic moieties of the acetal starting material. Also formed in lesser amounts is the trialkoxyethane of the general formula

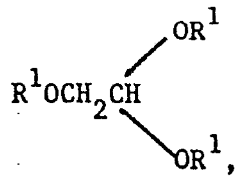

together with the alcohol $R^2OH$, which may be recycled to form additional acetal starting material. Again, as above, if the R groups of the acetal are different, a mixture of corresponding R-substituted compounds will result.

As shown below, the selectivities for the desired monoether over the trialkoxy by-product are in the ratio of from about 3:1 to as much as 10:1 or more.

In a preferred embodiment of this invention, the starting materials are preferably symmetrical acetals where the R groups are lower alkyl groups of 1 to 4 carbon atoms, thereby forming the corresponding ethylene glycol mono-lower alkyl ethers such as the monomethyl ether, the monoethyl ether, and the like.

Alternatively, the acetal may contain such $R^1$ and $R^2$ groups as naphthyl and phenyl. In the case of naphthyl, the reaction of the resulting formaldehyde acetal with syngas will provide 2-(2-naphthyloxy) ethanol, a known sedative, which in turn may be oxidized to the corresponding 2-naphthyloxyacetic acid, a plant growth hormone.

Likewise, the dealkoxyhydroxymethylation of the acetal, wherein $R^1$ and $R^2$ are phenyl, will produce 2-phenoxy-ethanol, a topical antiseptic, which when oxidized, results in phenoxyacetic acid, a fungicide. Similarly, the formaldehyde acetal wherein $R^1$ and $R^2$ are 2, 4, 5-trichlorophenyl will yield, in accordance with this process, 2, 4, 5-trichlorophenoxyacetic acid, an herbicide. In a like manner, when $R^1$ and $R^2$ are p-nonylphenyl, p-nonylphenoxyacetic acid, a corrosion inhibitor and antifoaming agent in gasoline and cutting oils will be formed.

Each of these aforedescribed products may be recovered routinely by methods well known in the art.

The invention will now be illustrated by, but is not intended to be limited to, the following examples.

## EXAMPLES

### Examples 1-21

A series of runs was carried out in which the following general procedure was employed, using as the catalyst a mixture of $Co_2(CO)_8$ and $Ru_3(CO)_{12}$, or $Co_2(CO)_8$ alone (for comparative purposes). In the table below, it should be noted that whereas addition of $Ru_3(CO)_{12}$ caused an increase in production of the glycolether, B, over that obtained with $CO_2(CO)_8$ alone, other metal carbonyls had no such effect.

To a 300 ml stainless steel autoclave equipped with a magnedrive stirrer was charged: methylal, solvent (if any), and catalyst. Carbon monoxide, 800 psig, and hydrogen, 1600 psig, were admitted and the reaction mixture was rapidly heated to the desired temperature. The mixture was stirred for the designated time at reaction temperature after which the reactor was cooled by immersion in an ice bath. When the contents reached 25°C the final pressure was recorded. After venting the gas the liquid was analyzed by GLPC.

The results are reported in Table I below. Reaction conditions, amounts, and the use of solvents were varied in accordance with the data set forth in this table.

TABLE I

ETHYLENE GLYCOL MONOMETHYL ETHER VIA DEALKOXYHYDROXYMETHYLATION OF METHYLAL

$$CH_2(OCH_3)_2 + CO + 2H_2 \longrightarrow [CH_3OCH_2CHO] \longrightarrow CH_3OCH_2CH_2OH + CH_3OH$$

(B)

$$\downarrow$$

$$[CH_3OCH_2CH(OCH_3)_2]$$

(A)

| Examples | $Co_2(CO)_8$ (gms.) | $Ru_3(CO)_{12}$ (gms.) | OTHER CATS. (2 gms.) | SOLVENT | METHYLAL Vol. % | T, °C | T, HRS. | AP | CONV. % | SELECTIVITY, % (A) | (B) |
|---|---|---|---|---|---|---|---|---|---|---|---|
| 1 | 2 | 0 | - | None | 100 | 110 | 4 | 1480 | 68 | 33 | 3 |
| 2 | 2 | 2 | - | None | 100 | 110 | 2.5 | 1358 | 77 | 19 | 16 |
| 3 | 2 | 0 | - | None | 100 | 150 | 1 | 1535 | 92 | 7 | 22 |
| 4 | 2 | 2 | - | None | 100 | 150 | 1 | 1617 | 88 | 7 | 37 |
| 5 | 2 | 0 | - | None | 100 | 150 | 2.5 | 1885 | 95 | 2 | 20 |
| 6 | 2 | 2 | - | None | 100 | 150 | 2.5 | 1721 | 95 | 7 | 37 |
| 7 | 1 | 2 | - | None | 100 | 150 | 4 | 1816 | 93 | 2 | 38 |
| 8 | 1 | 0 | - | None | 100 | 150 | 2.5 | 1847 | 89 | 2 | 18 |
| 9 | 1 | 2 | - | None | 100 | 150 | 2.5 | 1840 | 87 | 3 | 34 |
| 10 | 1 | 0 | $Os_3(CO)_{12}$ | None | 100 | 150 | 1 | 1410 | 81 | 7 | 19 |
| 11 | 1 | 0 | $Fe(CO)_5$ | None | 100 | 150 | 1 | 1310 | 81 | 7 | 16 |
| 12 | 1 | 0 | $Ir_4(CO)_{12}$ | None | 100 | 150 | 1 | 1469 | 79 | 7 | 15 |
| 13 | 1 | 0 | $Re_2(CO)_{10}$ | None | 100 | 150 | 1 | 1274 | 70 | 16 | 13 |
| 14 | 1 | 0 | $Cr(CO)_6$ | None | 100 | 150 | 1 | 1439 | 73 | 10 | 13 |
| 15 | 1 | 0 | $Fe_3(CO)_{12}$ | None | 100 | 150 | 1 | 1310 | 79 | 6 | 13 |
| 16 | 1 | 0 | $RhCl_3$ | None | 100 | 150 | 4 | 916 | 57 | 12 | 4 |
| 17 | 1 | 0 | - | THF(1) | 45 | 150 | 2 | 1252 | 96 | 2 | 19 |
| 18 | 1 | 2 | - | THF | 45 | 150 | 4 | 1257 | >99 | 1 | 40 |
| 19 | 1 | 0 | - | Toluene | 45 | 150 | 3 | 707 | 76 | 7 | 24 |
| 20 | 1 | 0 | - | DMF(2) | 45 | 150 | 4 | 116 | 74 | TR | 0.3 |
| 21 | 0 | 2 | - | None | 100 | 150 | 2.5 | 106 | 1 | 1 | 0.2 |

(1) THF = tetrahydrofuran
(2) DMF = dimethylformamide

Examples 22-25

In addition to the above runs, another series of runs was carried out in accordance with the following general procedure, using varying conditions, etc., in which the catalyst employed was precipitated from solutions of Co-Ru catalyzed methylal dealkoxyhydroxymethylation, with or without a solvent being present.

The catalyst system and methylal were charged to a 300 ml autoclave and the system flushed thoroughly with CO and $H_2$. A 1:1 $CO/H_2$ mixture was added to a pressure of 2400 psig and the temperature rapidly raised to 150°C. The reaction mixture was stirred for the designated time period and then rapidly cooled, the pressure released, gases collected and analyzed by gcms and the liquid removed and analyzed by standardized glpc.

The results of these runs are reported in Table II below.

TABLE II

CATALYTIC DEALKOXYHYDROXYMETHYLATION OF METHYLAL USING COBALT-RUTHENIUM CARBONYLS

| EXAMPLE | $Co_2(CO)_8$ (gms.) | $Ru_3(CO)_{12}$ (gms.) | OTHER, gms. | METHYLAL gms. | $P_{total}$ (f) psig | $CO/H_2$ Ratio | T °C | T HRS. | P psig | SELECTIVITY TO $C_2$ % (a) | $C_2$ Ratio (b) | METHYLAL CONV., % |
|---|---|---|---|---|---|---|---|---|---|---|---|---|
| 22 | 1.5 | 3.0 | - - | 71.4 | 2445 | 1/2 | 150 | 2.0 | 1980 | 45.4 | 9.0 | 70.1 |
| 23 | - | - | (c), 2.5 | 59.3 | 2430 | 1/2 | 150 | 3.0 | 1813 | 55.3 | 11.8 | 73.7 |
| 24 | - | - | (d), 2.0 | 47.3 | 2415 | 1/2 | 150 | 4.0 | 1171 | 67.2 | 3.7 | 64.6 |
| 25 | - | - | (e), 18.4 | 71.4 | 2432 | 1/2 | 150 | 3.0 | 1918 | 65.2 | 7.4 | 60.4 |

a) Selectivity = (moles of $CH_3OCH_2CH_2OH$ + $CH_3OCH_2CH(OCH_3)_2$ formed/moles of $CH_2(OCH_3)_2$ reacted).

b) $C_2$ ratio = moles of $CH_3OCH_2CH_2OH$/moles of $CH_3OCH_2CH(OCH_3)_2$.

c) Catalyst isolated by precipitation from solutions of Co-Ru catalyzed methylal dealkoxyhydroxymethylations in which THF was used as the solvent. (See Examples 23 catalyst preparation below.)

d) Catalyst isolated by precipitation from solutions of Co-Ru catalyzed methylal dealkoxylhydroxymethylations carried out in neat methylal. (See Example 24 catalyst preparation below.)

e) Catalyst prepared by heating a mixture of $[Co_2(CO)_8]$, 2 gms. and $[Ru_3(CO)_{12}]$, 4 gms. in chlorobenzene at 150°C under 3000 psi of syngas ($CO/H_2$ = 1/2) for 3 hrs. On cooling 2 gms. of brick-orange solid precipitated (See Example 25 catalyst preparation below.)

f) $P_{Total}$ = total pressure uptake during reaction at 150°C (in psig)

BAD ORIGINAL

0168989

The catalysts for Examples 23, 24 and 25 of Table II were prepared as follows:

## Example 23 Catalyst

The catalyst for Example 23 was prepared by allowing the liquid recovered from two runs identical to Example 18, Table 1 to stand for several weeks, after which an orange solid precipitated. Filtration of the orange solid followed by drying in vacuo gave a material which exhibited characteristic metal carbonyl bands in the infrared but was neither $Co_2(CO)_8$ nor $Ru_3(CO)_{12}$. The ir bands in the isolated solid were at 4.83, 4.92 and 4.98u. The bridging carbonyl of the starting $Co_2(CO)_8$ (5.37u) had totally disappeared in forming the new active complex. The solid, 2.5 grams, was used as a catalyst for Example 23.

## Example 24 Catalyst

The catalyst for Example 24 was prepared by allowing the liquid recovered from two runs identical to Example 4, Table 1 to stand for several weeks after which an orange solid precipitated. This solid, 2.0 grams, after filtration and drying in vacuo, was used as the catalyst for Example 24. The solid which was isolated had characteristic metal carbonyl bands at 4.83, 4.92 and 4.98u. The bridging carbonyl of the starting $Co_2(CO)_8$ had completely disappeared.

Example 25 Catalyst

The catalyst for Example 25 was prepared as indicated in footnote (c) of Table II. The isolated orange solid had infrared bands at 4.83u, 4.92u an 4.97u indicative of a metal carbonyl complex in which there was no bridging carbonyl band at 5.37u.

In the following examples, Examples 26-32, experiments were carried out in a manner similar to Examples 1-25, except that dibutoxymethane was substituted for methylal, and solvents and temperatures were varied as shown in Tables III and IV, to produce ethylene glycol monobutyl ether.

## Examples 26-29

In Examples 26-29 formaldehyde dibutylacetal, ($CH_2(OBu)_2$), was dealkoxylhydroxymethylated to ethylene glycol monobutyl ether, EGMBE, using a 1:1 mixture of $Co_2(CO)_8$ and $Ru_3(CO)_{12}$ as the catalyst. It can be seen from the tabulated results of Table III that running the reaction in a solvent such as n-butyl alcohol or o-dichlorobenzene gives generally superior yields to running the reaction in neat $CH_2(OBu)_2$.

In Table III, the examples were conducted in accordance with the following general procedure:

Dicobalt octacarbonyl, 4 mmoles, triruthenium dodecacarbonyl, 4 mmoles, $CH_2(OBu)_2$ in the amount listed in the table and solvent in the amount listed in the table were charged to a 300 ml stirred autoclave to which 3200 psi of a 1:1 mixture of CO and $H_2$ were added and then heated to 150°C for 3-4 hrs. The autoclave was cooled, drained and the product weighed and subjected to standardized gas chromatographic analysis.

TABLE III

EFFECTS OF SOLVENTS ON DEALKOXYHYDROXYMETHYLATION OF BUTYLAL

| Example | Solvents | Ml. | $CH_2(OBu)_2$ Used, Ml. | G. C. Analysis Reaction Mixture, Area %: $CH_3OBu$ | $PhCH_3$ (a) | BuOH | $CH_2(OBu)_2$ | EGMBE (b) | TBE (c) | Unidentified Heavies | Solvent | Conv. of $CH_2(OBu)_2$ Mole % | Selectivity to EGMBE Mole % | (d) Yield of EGMBE Mole % |
|---|---|---|---|---|---|---|---|---|---|---|---|---|---|---|
| 26 | None | 0 | 90 | 6.0 | 19.1 | 33.3 | 6.7 | 18.8 | 4.3 | 7.3 | | 94 | 32 | 30 |
| 27 | n-BuOH | 45 | 45 | 2.5 | 17.2 | 61.6 | trace | 13.7 | 3.2 | 1.8 | | >99 | 44 | 44 |
| 28 | n-BuOH | 67 | 23 | 0.9 | 16.4 | 73.8 | trace | 4.8 | 1.0 | 3.2 | | >99 | 28 | 28 (e) |
| 29 | $o-Cl_2C_6H_4$ (f) | 67 | 23 | 0.9 | 18.0 | 5.7 | trace | 5.0 | 0.3 | 0.9 | 69.1 | >99 | 42 | 42 |

a) $PhCH_3$ is the internal standard

b) EGMBE - ethylene glycol monobutyl ether

c) TBE - 1,1,2-tributoxyethane

d) [(mmoles EGMBE in recovered liquid)/(mmoles $CH_2(OBu)_2$ charged)]x100

e) low product yield due to mechanical losses

f) $o-Cl_2C_6H_4$ - o-dichlorobenzene

Examples 30-32

In Examples 30-32, the results of which are summarized in Table IV, it can be seen that as the reaction temperature is raised from 125 to 175°C the EGMBE yield increases from 12 to 34.

In Table IV, the examples were conducted in accordance with the following general procedure:

Dicobalt octacarbonyl, 4 mmoles, triruthenium dodecacarbonyl, 4 mmoles, and $CH_2(OBu)_2$, 90 ml, were charged to a 300 ml autoclave. Then 3200 psi of a 1:1 $CO/H_2$ mixture was added and the reaction mixture heated for 3-4 hours at the temperature listed in the Table. The autoclave was cooled, drained and the product weighed and subjected to standardized gas chromatographic analysis.

TABLE IV

EFFECTS OF TEMPERATURE ON DEALKOXYHYDROXYMETHYLATION OF BUTYLAL

| Exam. | $[Co_2(CO)_8]$ mmoles | T, °C | $CH_2(OBu)_2$ Used, Ml. | G. C. Analysis Reaction Mixture, Area %: $CH_3OBu$ | $PhCH_3$ (a) | BuOH | $CH_2(OBu)_2$ | EGMBE (b) | TBE (c) | Unidentified Heavies | Conv. of $CH_2(OBu)_2$ Mole % | Selectivity to EGMBE Mole % | (d) Yield of EGMBE Mole % |
|---|---|---|---|---|---|---|---|---|---|---|---|---|---|
| 30 | 4 | 125 | 90 | 1.8 | 17.0 | 20.1 | 30.5 | 7.1 | 10.4 | 10.6 | 69 | 17 | 12 |
| 31 | 4 | 150 | 90 | 6.0 | 19.1 | 33.3 | 6.7 | 18.8 | 4.3 | 7.3 | 94 | 32 | 30 |
| 32 | 4 | 175 | 90 | 11.1 | 18.3 | 35.0 | 3.3 | 20.8 | 2.6 | 3.3 | 97 | 35 | 34 |

a) $PhCH_3$ is the internal standard

b) EGMBE - ethyleneglycol monobutyl ether

c) TBE - 1,1,2-tributoxyethane

d) [(mmoles EGMBE in recovered liquid)/(mmoles of $CH_2(OBu)_2$ charged)]x100

### Example 33

In a manner similar to Example 31, except that formaldehyde dicyclohexyl acetal is used instead of formaldehyde dibutyl acetal, ethylene glycol monocyclohexyl ether is prepared in good yield.

### Example 34

In a manner similar to Example 31, except that formaldehyde diphenyl acetal is used instead of formaldehyde dibutyl acetal, ethylene glycol monophenyl ether is formed as a reaction product.

### Example 35

In a manner similar to Example 31, except that dioxolane is used instead of formaldehyde dibutyl acetal, diethylene glycol is produced as a reaction product.

### Example 36-43

Approximately 6 grams of paraformaldehyde and the catalyst package in 90 moles of butanol and 10 ml of toluene were heated at the temperatures given in Table V at the pressures of hydrogen and CO indicated for two hours. After this time the reaction mixtures were analyzed by gas chromatography showing the major product to be ethylene glycol monobutyl ether. Results are tabulated in Table V.

TABLE V

REACTIONS OF FORMALDEHYDE, SYNGAS AND BUTANOL

TO GIVE ETHYLENEGLYCOL MONOBUTYL ETHER, EGMBE

| EXAMPLE | CATALYST $Co_2(CO)_8$ | COMPONENTS $Ru_3(CO)_{12}$ | INITIAL $H_2$ | PRESSURES CO | REACTION T. °C | REACTION PRODUCTS (GC Area % $CH_3OC_5H_9$ | EGMBE | $CH_2(OC_4H_9)$ |
|---|---|---|---|---|---|---|---|---|
| 36 | 4 | 4 | 1600 | 800 | 150 | 2.0 | 9.4 | tr |
| 37 | 4 | 4 | 1600 | 800 | 150 | 2.0 | 9.7 | 0.3 |
| 38 | 2 | 4 | 1600 | 800 | 150 | 1.7 | 9.6 | 0.5 |
| 39 | 2 | 4 | 1600 | 800 | 150 | 1.4 | 9.1 | 1.5 |
| 40 | 4 | 4 | 1600 | 1600 | 150 | 1.2 | 10.7 | 0.5 |
| 41 | 4 | 4 | 1600 | 1600 | 150 | 0.7 | 9.2 | 1.8 |
| 42 | 2 | 4 | 1600 | 1600 | 150 | 0.7 | 9.2 | 1.8 |
| 43 | 4 | 4 | 1600 | 800 | 175 | 2.3 | 9.6 | - |

a) EGMB = Ethylene glycol monobutyl ether

BAD ORIGINAL

Examples 44-50

A series of runs was carried out in which the following general procedure was employed, using as the catalyst $Co_2Ru(CO)_{11}$ as well as mixtures of $Co_2(CO)_8$ and $Ru_3(CO)_{12}$, or $Co_2(CO)_8$ alone for comparative purposes:

To a 300 ml stainless steel autoclave equipped with a magnedrive stirrer was charged: methylal, solvent and catalyst. Carbon monoxide and hydrogen were admitted and the reaction mixture was rapidly heated to the desired temperature. The mixture was stirred for the designated time at reaction temperature after which the reactor was cooled by immersion in an ice bath. When the contents reached 25°C, the final pressure was recorded. After venting the gas, the liquid was analyzed by GLPC.

The results are reported in Table VI below. The specific reaction conditions, amounts, and solvent are as described in footnote (a) in this table.

TABLE VI

METHYLAL DEALKOXHYDROXYMETHYLATION(a)

| Examples | Catalyst Used, mmoles $[Co_2(CO)_8]$ | $[Ru_3(CO)_{12}]$ | $[RuCo_2(CO)_{11}]$ | $CH_2(OCH_3)_2$ | GC Analysis of Reaction Mixture $CH_3OH$ | EGMME(b) | OTHERS(c) | Conv. Wt. % | Wt. % EGMME(b) In Reaction Product(d) |
|---|---|---|---|---|---|---|---|---|---|
| 44 | 5 | 0 | 0 | 25 | 42 | 18 | 15 | 75 | 24 |
| 45 | 5 | 5 | 0 | 29 | 33 | 30 | 8 | 71 | 42 |
| 46 | 3 | 0 | 0 | 33 | 33 | 17 | 17 | 67 | 25 |
| 47 | 3 | 1 | 0 | 37 | 26 | 25 | 12 | 63 | 40 |
| 48 | 3 | 3 | 0 | 38 | 30 | 25 | 7 | 62 | 40 |
| 49 | 0 | 0 | 3 | 36 | 31 | 22 | 11 | 64 | 34 |
| 50 | 0 | 0 | 3 | 36 | 29 | 24 | 11 | 64 | 38 |

(a) A mole of methylal, .094 moles of toluene, and the catalyst(s) were pressured to 2400 psig with a 2/1 mixture of $H_2/CO$ and heated for 2-3 hrs.

at 150°C. The reaction mixture was analyzed by GC.

(b) EGGME = $CH_3OCH_2CH_2OH$ + related $C_2$ products (i.e., $CH_3OCH_2CH(OCH_3)_2$)

(c) Others = $CH_3OCH_3$, $CH_3CH_2OH$ and heavy by-products

(d) [EGGME/($CH_3OH$ + EGMME + Others)] x 100

Example 51

To a 110 ml. rocking autoclave are charged $Co_2Ru(CO)_{11}$, (0.75 mmoles); methylal (70.6 mmoles); and o-dichlorobenzene, (23.55 gms). After flushing thoroughly with 1/2 ($CO:H_2$) syngas, 800 psig of CO is admitted, then hydrogen to a total pressure of 2400 psig. The autoclave is rocked at 150°C for 3 hours, then cooled, the product removed and analyzed by standardizing GLPC. A high yield of ethylene glycol monomethyl ether is obtained.

Example 52

Using the procedures of Example 51, but substituting diethoxymethane for methylal, a good yield of ethylene glycol monoethyl ether is obtained.

Example 53

Using the procedures of Example 51, but substituting dibutoxymethane for methylal, an excellent yield of ethylene glycol monobutyl ether is obtained.

Example 54

Using the procedures of Example 51, but substituting 0.4 mmoles of paraformaldehyde for methylal, and 25 grams of butanol for o-dichlorobenzene, a good yield of ethylene glycol monobutyl ether is obtained.

0168989

Example 55

Using the procedure of Example 51 but substituting diethoxyethane for methylal, propylene glycol monoethyl ether is the major reaction product.

Example 56

To a 110 ml. rocking autoclave is charged $Co_2Ru(CO)_{11}$ (0.75 mmoles); methylal (27 mmole), butanol (18.5 ml.), and mesitylene as an internal standard. Carbon monoxide, 800 psig, is added and hydrogen added to a total pressure of 3200 psig. The mixture is rocked at 150°C for six hours, cooled and analyzed by standardized GLPC. A good yield of the monobutylether of ethylene glycol is produced.

CLAIMS:

1. A process for the dealkoxyhydroxymethylation of a formaldehyde acetal of the formula

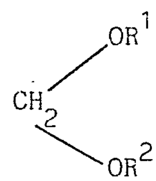

wherein $R^1$ and $R^2$, which may be same or different, are selected from alkyl, cycloalkyl, and aryl moieties having from 1 to 20 carbon atoms, and wherein $R^1$ and $R^2$, taken together, may be joined to form a cyclic acetal,

which comprises reacting said acetal, which has been formed separately or in situ, with syngas in the presence of a catalytically effective amount of a catalyst comprising a mixture of $Co_2(CO)_8$ promoted with $Ru_3(CO)_{12}$ to form the corresponding ethylene glycol monoëther.

2. A process according to claim 1, wherein the molar ratio of cobalt to ruthenium carbonyl components of the catalyst mixture is in the range of from 10:1 to 1:10.

3. A process according to claim 1 or 2, wherein the temperature is in the range of from 100 to 200°C.

4. A process according to any of claims 1 to 3, wherein the pressure is in the range of from 34 to 345 bar (500 to 5000 psi).

5. A process according to any of claims 1 to 4, further comprising carrying out the reaction in the presence of an inert organic solvent.

6. A process according to claim 5, wherein the inert organic solvent is a chlorinated aromatic solvent.

BAD ORIGINAL

7. A process according to claim 6, wherein the chlorinated aromatic solvent is chlorobenzene or dichlorobenzene.

8. A process according to any of claims 1 to 7, wherein $R^1$ and $R^2$ are alkyl groups having from 1 to 20 carbon atoms.

9. A process according to claim 8, wherein the alkyl groups are lower alkyl.

10. A process according to any of claims 1 to 9, wherein the weight ratio of catalyst to acetal is in the range of from 0.001 to 0.1.

11. A composition useful as a catalyst for the dealkoxyhydroxymethylation of acetals comprising a mixture of $Co_2(CO)_8$ promoted with $Ru_3(CO)_{12}$.

12. A composition according to Claim 11, wherein the molar ratio of the cobalt to ruthenium carbonyl components of the catalyst mixture is in the range of from 10:1 to 1:10.

13. A composition useful as a catalyst for the dealkoxyhydroxymethylation of acetals prepared by precipitating a ruthenium and cobalt carbonyl complex from a reaction mixture formed by the dealkoxyhydroxymethylation of a formaldehyde acetal with syngas in the presence of a catalyst comprising a mixture of $Co_2(CO)_8$ promoted with $Ru_3(CO)_{12}$.

14. A composition useful as a catalyst for the dealkoxyhydroxymethylation of acetals prepared by precipitating a ruthenium and cobalt carbonyl complex from a reaction mixture formed by the dealkoxyhydroxymethylation of a formaldehyde acetal with syngas in the presence of an inert organic solvent and a catalyst comprising a mixture of $Co_2(CO)_8$ promoted with $Ru_3(CO)_{12}$.

BAD ORIGINAL

15. A composition useful as a catalyst for the dealkoxyhydroxymethylation of acetals prepared by precipitating a ruthenium and cobalt carbonyl complex from a reaction mixture formed by the treatment of a mixture of $Co_2(CO)_8$ and $Ru_3(CO)_{12}$ in a chlorinated aromatic hydrocarbon with syngas at elevated temperature and pressure.

16. A composition according to any of claims 13 to 15, wherein the molar ratio of cobalt to ruthenium carbonyl components of the precipitated catalyst is in the range of from 10:1 to 1:10.

17. A process for the dealkoxyhydroxymethylation of an aldehyde acetal which comprises reacting said acetal, which has been formed separately or in situ, with syngas in the presence of a catalytically effective amount of a catalyst comprising the complex $Co_2Ru(CO)_{11}$ to form the corresponding glycol monoether.

18. A process according to claim 17 wherein the temperature is in the range of from 100 to 200$^{\circ}$C.

19. A process according to claim 17 or 18, wherein the pressure is in the range of from 34 to 345 bar (500 to 5000 psi).

20. A process according to any of claims 17 to 19, further comprising carrying out the reaction in the presence of an inert organic solvent.

21. A process according to claim 20, wherein the inert organic solvent is a chlorinated aromatic solvent.

22. A process according to claim 21, wherein the chlorinated aromatic solvent is chlorobenzene or dichlorobenzene.

23. A process according to any of claims 17 to 22, wherein the weight ratio of catalyst to acetal is in the range of from .001 to 0.1.

BAD ORIGINAL

0168989

European Patent Office

# EUROPEAN SEARCH REPORT

Application number

EP 85 30 4437

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | US-A-4 390 734 (J.F. KNIFTON) * Claims; example III * ----- | 1-12 | C 07 C 43/13<br>C 07 C 41/01<br>B 01 J 31/20 |
| | | | **TECHNICAL FIELDS SEARCHED (Int. Cl.4)** |
| | | | C 07 C 41/00<br>C 07 C 43/00<br>B 01 J 31/00 |

The present search report has been drawn up for all claims

| Place of search | Date of completion of the search | Examiner |
|---|---|---|
| THE HAGUE | 03-10-1985 | WRIGHT M.W. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document